# EUROPEAN PATENT SPECIFICATION

(11) **EP 1 123 701 B1**
(45) Date of publication and mention of the grant of the patent: **09.11.2005**
(21) Application number: 99938502.4
(22) Date of filing: 18.08.1999
(51) Int. Cl.: A61K 31/20, A61K 31/23, A61K 31/16, A23K 1/16, A23K 1/18, A61P 33/00

(54) **USE OF NATURAL PHYSIOLOGICALLY ACTIVE SUBSTANCES AGAINST FISH PARASITIC DISEASES**
VERWENDUNG NATÜRLICHER PHYSIOLOGISCH AKTIVER SUBSTANZEN GEGEN PARASITÄRE FISCHERKRANKUNGEN
UTILISATION DES SUBSTANCES NATURELLES PHYSIOLOGIQUEMENT ACTIVES CONTRE DES MALADIES PARASITAIRES DES POISSONS

(30) Priority: 25.08.1998 JP 23920298
(43) Date of publication of application: 16.08.2001
(73) Proprietor: NIPPON SUISAN KAISHA, LTD., Tokyo 100-0004 (JP)
(72) Inventor: HIRAZAWA, Noritaka, Nippon Suisan Kaisha, Ltd., Minamikaifu-gun, Oita 876-120 (JP); OSHIMA, Shunichiro, Nippon Suisan Kaisha, Ltd., Minamikaifu-gun, Oita 876-120 (JP); MITSUBOSHI, Toru, Nippon Suisan Kaisha, Ltd., Minamikaifu-gun, Oita 876-1204 (JP); HATA, Kazuhiko, Nippon Suisan Kaisha, Ltd., Minamikaifu-gun, Oita 876-1204 (JP)
(74) Representative: TBK-Patent
(86) International application number: PCT/JP1999/004427
(87) International publication number: WO 2000/010558

(56) References cited:
- JP-A- 4 158 750
- US-A- 5 208 257
- AKAO N ET AL: "IN VITRO ASSESSMENT OF MORBIDITY OF TOXOCARA CANIS LARVAE USING A DYE EXLUSION ASSAY" KISEICHUGAKU ZASSHI - JAPANESE JOURNAL OF PARASITOLOGY, MATSUBAYASHI, TOKYO, JP, vol. 41, no. 6, December 1992 (1992-12), pages S19-S26, XP000917445 ISSN: 0021-5171
- FUMIYUKI KIUCHI ET AL.: "Studies on crude drugs effective on visceral larva migrans. III. The bursting activity of tannins on dog roundworm larva" CHEMICAL AND PHARMACEUTICAL BULLETIN., vol. 36, no. 5, 1988, pages 1796-1802, XP002241479 PHARMACEUTICAL SOCIETY OF JAPAN. TOKYO., JP ISSN: 0009-2363

## Description

### Technical Field

The present invention relates to the use of a natural physiologically active material effective on fish diseases and fish food containing the material. More particularly, the present invention relates to the use of fatty acids having carbon numbers 6 to 12 which effectively act on fish parasitic diseases, and fish food containing at least one of the fatty acids.

### Background Art

In aquaculture, fish diseases are very serious problems because of their great effects on stable production of cultured fish. Bacterial diseases are treated in the field by applying antibiotic substances. With application of antibiotic substances, however, there emerge resistant bacteria against the antibiotic substances used, resulting in a difficulty in ensuring stable production of cultured fish and giving rise to environmental issues.

Also, recently, virus diseases have infected major cultured fish in many cases and have become serious problems in the aquaculture industry. At present, there are no effective chemotherapeutic agents for the virus diseases, and development of vaccine is delayed.

Further, parasitic diseases posing serious problems are the monogenean *Heterobothrium okamotoi* inflection in tiger puffer *Tkifugu* *rubripes* and the skin parasite disease in yellowtail *Seriora quinqueradiata*. The *Heterobothrium okamotoi* infection has been treated in the field just by routinely immersing fish in a drug bath for about one hour in which about 800 ppm of formalin is dissolved. However, this treatment can merely destroy about 80 % of larvae at maximum, and cannot destroy adult parasites at all. Also, formalin is a carcinogenic substance and arise a problem from the viewpoint of environmental pollution. No effective preventable measures are known for the skin parasite disease. Skin parasites can be completely destroyed by immersing fish in a freshwater bath for about five minutes, or by applying a commercially available anthelmintic which contains hydrogen peroxide as a main ingredient. Replacing nets routinely is also effective to remove eggs attached to the nets. It would be difficult to reduce an infection level in the fish-culturing farm unless the farm is entirely treated at a time. Also, since eggs attached to the net filaments are survived after the treatment for destroying parasites, it is actually needed to repeat the treatment routinely. Affinitive species, which have brought into Japan with amberjack *Seriora dumerili* imported from China and Hongkong in 1990s, have low host peculiarity and spread into not only amberjack but also many other kinds of fish, including yellowtail, cultivated in sea.

For those reasons, development of a natural material effective on fish diseases when used by oral administration, and proper treatment for fish diseases using such a natural material is demanded.

### DISCLOSURE OF THE INVENTION

An object of the present invention is to provide the use of a natural physiologically active material effective on parasitic diseases, when used by oral administration, and fish food containing the material.

The inventors have made screening of various natural materials with a view of developing a natural material which is effective to fish diseases when put into food for oral. As a result, the inventors have found that fatty acids having carbon number 6 to 12 effectively act on any of bacteria, viruses and parasites causing fish diseases, and the use of such fatty acids by oral administration remedies the fish diseases and prevents their occurrence. Based on this finding, the present invention has been accomplished.

The fact that medium-chain fatty acids have an antimicrobial activity and an antivirus activity is in itself known. In the past, however, those activities have been demonstrated by tests just contacting those fatty acids with bacteria and viruses at *in vitro* level, and the efficacy of those fatty acids resulted through oral administration at in vivo level has not yet been reported, except Japanese Patent No.2831835, which discribes actions of fatty acids against bacteria in vivo. Actions of those fatty acids on parasites causing fish disease are not yet known. No reports have been publicized regarding the cases of adding those fatty acids in fish food and preventing and remedying the fish diseases causing by parasites. Examples of previous related reports are as follows. (All of these reports are based on *in vitro* experiments.)

Lett. Appl. Microbiol, 27(6)362-868(1988) describes a bactericidal action of lauric acid against Gram-positive bacteria (*Lysteria monocytogenes*). Antimicrob. Agents Chemother, 40(2)302-306(1996) describes actions of medium-chain fatty acids against Helicobacter pylori. US 4002775 describes an antibacterial activity of fatty acids. Arch. Virol. 66(4)301-307(1980) describes actions of unsaturated fatty acids against enveloped viruses in animals at *in vitro* level. EP 465423 describes actions of fatty acids against bacteria and viruses. Pstic. Sci. 30, 295-202(1990) describes actions of fatty acids against harmful insects on plants (cowpea *Vigna catiang*). Pesticide Biochem. Physiol. 50, 229-239(1994) describes of fatty acids against nematodes on plants. EP 279523 describes a pesticidal action (against lice) of fatty acids. Antimicrob. Agents Chemother, 31(1)27-31(1987) describes actions of medium-chain fatty acids against enveloped viruses. Chem. Pharm. Bull. 35(7)2880-2886(1987) describes actions of fatty acids against dog's parasites. Fish Pathology, 32(1)15-20(1997) describes the *Heterobothrium okamotoi* infection in tiger puffer. Fish Pathology, 33(4)221-227(1998) describes bacterial diseases of cultured fish. Fish Pathology, 33(4)303-309(1998) describes parasitic diseases of cultured fish. Gyobyo Kenkyu, 27(2)97-102(1992) describes Kuchijirosho (snout ulcer disease) in tiger puffer.

US-A-5 208 257 is directed to an anti-parasite pharmaceutical composition for the tropical application to human or lower animals preventing microbial related skin conditions. This composition contains C₆ to C₁₈ fatty acids (cf. abstract, claim 1).

AKAO N, et al., Jpn. J. Parasitol., Vol. 41, No. 6, p. 519-526 and KIUCHI F, et al., Chem. Pharm. Bull., Vol. 36, p. 1796-1802 describe *in vitro* experiments on the larvicidal activity of capric acid.

JP-A-4158750 discloses a fish food which inhibits the growth of viral or bacterial pathogens causing infectious diseases. This feed may contain a C6-12 medium-chain fatty acid, e.g. caproic acid, caprylic acid or lauric acid, or their salts.

WO 98/23152 discloses compositions containing a material having a vitamin C activity (i.e. an ascorbic acid derivative in the form of a fatty acid ester) which may comprises capric glyceride or caprylic glyceride as a solvent (cf. e.g. page 11, 2^{nd} paragraph).

JP-A-1-296953 discloses a feed for aquatic organisms, wherein vitamin C particles are coated with a composition, which may comprises capric acid.

JP-A-6-336443 describes a composition containing L-ascorbic acid ester and caprylic acid/capric acid triglycerides, which composition may be used for food or pharmaceuticals.

The results that fatty acids having carbon number 6 to 12 effectively act on any of fish diseases attributable to bacteria, viruses and parasites, and that the use of such fatty acids by oral administration remedies the fish diseases and prevents their occurrence, are unique discovery made by the inventors. The present invention has been accomplished with intensive studies based on that discovery.

More specifically, one aspect of the present invention resides in the use of a natural physiologically active material defined according to claim 1.

When fish diseases are parasitic diseases, the present invention provides the use of a natural physiologically active material effective on the parasitic diseases, which contains at least one of fatty acids having carbon number 6 to 12 and, as required, a material having a vitamin C activity.

The fatty acids having carbon number 6 to 12 include, e.g., caproic acid (C6), caprylic acid (C8), pelargonic acid (C9), capric acid (C10), and lauric acid (C12). Preferable fatty acids having carbon number 6 to 12 are capric acid, caprylic acid and pelargonic acid. In this case, the present invention provides a natural physiologically active material effective fish diseases, specifically parasitic diseases, bacterial diseases and/or virus diseases, which contains at least one of capric acid, caprylic acid and pelargonic acid. Another aspect of the present invention resides in the use of a formulation as fish food containing a natural physiologically active material effective on fish parasitic diseases, which contains at least one of fatty acids having carbon number 6 to 12, specifically capric acid, caprylic acid and pelargonic acid. The content of the natural physiologically active material is preferably approximately 0.25 weight % that is suitable in providing a proper effect on the parasitic diseases.
Still another aspect of the present invention resides in the use of a formulation as an food additive containing a natural physiologically active material effective on fish diseases, specifically parasitic diseases, bacterial diseases and virus diseases, which contains at least one of fatty acids having carbon number 6 to 12, specifically capric acid, caprylic acid and pelargonic acid. The content of the natural physiologically active material is preferably approximately 0.25 weight % that is suitable in providing a proper effect on the parasitic diseases.

The natural physiologically active material of the present invention is a material which contains at least one of fatty acids having carbon number 6 to 12, is effective on fish diseases, and acts on only fish parasites, harmful to fish. These results are proved from Examples described later along with the fact that the natural physiologically active material of the present invention is effective on many known parasitic diseases of fish; adequate concentrations of the material to be applied for respective kinds of fish; and the fact that the efficacy of the fatty acids is enhanced by a material having a vitamin C activity is used in a combined manner as required.
The present invention is applicable to all kinds of cultured fish. Examples of the cultured fish include puffers (tiger puffer), sea breams (red sea bream *Pagrus major* and *black* porgy *Acanthopagrus schlegeli*), amberfish (yellowtail, striped amberjack *Seriola lalanndi*, amberjack, and long-fin amberjack *Seriola rivoliana*), salmons (rainbow trount and coho salmon), plaices (founder and spotted plaice), and carps (cyprinid *Cyprinus* and cyprinoid *Ctenopharyngodonib idellus*).
Fish parasitic diseases will be described below. Tiger puffer is affected by, e.g., the *Heterobothrium okamotoi* infection and the *Cryptocaryon irritans* infection. Red seam bream is affected by, e.g., the *Cryptocaryon irritans* infection and the *Bivagina* tai infection. Flounder is affected by, e.g., the *Cryptocaryon irritans* infection. Amberjack is infected, e.g., the blood fluke(Paradeontacylix) infection and the *Heteraxine heterocerca* infection. The life cycle of the pathogen of the *Heterobothrium okamotoi* infection of tiger puffer is below. The oncomiracidia hatched from eggs first attach to the gill and grow there to larvae. The larvae having grown to about 5 - 6 mm move to the branchial cavity wall, and become matured parasites with their grippers imbedded in the tissue. The matured parasites then lay eggs in the tissue. The pathogen of the skin parasite disease of yellowtail is Benedenia serilae belonging to monogenean *Copsala Benedenia*, and lives in striped amberjack, amberjack and long-fin amberjack in addition to yellowtail. No natural materials effective on those fish diseases and being usable in the actual culture farm have been known.

The inventors previously elucidated that pranziquantel used as a parasiticide against liver trematode in human beings is effective against a fish parasite *Heterobothrium okamotoi*, and filed a patent application (Japanese Patent Application No. 9-230316). Also, lactoferrin is well known as multifunctional protein contained in mother's milk. For mammals, it is suggested that lactoferrin takes part in local prevention against infection as found with lysozyme and secretor IgA. For fish, it is reported that lactoferrin derived from mammals shows immunological enhancement against bacterial infection in rainbow trout when used by oral administration. Of late, the inventors elucidated that lactoferrin is effective on both mammals and fish in common, and filed a patent application for fish food which contains lactoferrin as a natural physiologically active material effective against *Heterobothrium okamotoi* (Japanese Patent Application No. 9-230317).

In the art of fish culture, development of drugs and natural materials, which are effective against parasites at low concentrations, is very important for the purpose of reliably eliminating ectoparasites on fish. In particular, there is a keen demand for development of a natural material like lactoferrin which has been proved to be effective against the parasitic diseases in common to both mammals and fish.

Fish bacterial diseases will be described below. To develop a natural material, which is effective on the fish bacterial diseases when used by oral administration in the culture farm, without resorting to antibiotic substances is keenly demanded from the viewpoints of not only stable production of fish but also protection of environments. Responsive to such a demand, the inventors studied the antibacterial activity of fatty acids, mainly decanoic acid, on the fish bacterial diseases (*in vitro* test against fish disease pathogens), and found that medium-chain fatty acids have an antibacterial activity. Bacteria causing the fish bacterial diseases include, for example, *Vibrio, Edwardsiella* and enterococcus, but not limited to these species.

Fish virus diseases will be described. The inventors obtained the result that oral administration of fatty acids having carbon number 6 to 12 showed the efficacy in preventing the occurrence of the virus diseases in fish. Viruses causing the fish virus diseases include, e.g., Kuchijirosho virus in tiger puffer, but not limited to this species.

The fatty acids used in the present invention are fatty acids having carbon number 6 to 12. These fatty acids are preferably free fatty acids, but are not limited to free fatty acids and also effective in the forms of salts, glycerides, esters and amides having residual radicals of the fatty acids having carbon number 6 to 12 in their molecules. An example of salts is sodium salt. Examples of glycerides include monoglycerides, diglycerides, and triglycerides. Examples of esters include ethylesters and methylesters. Examples of amides include ethylamides and methylamides.

Examples of the material having a vitamin C activity include ascorbic acid, sodium ascorbate, and ascorbate esters.

Octanoic acid as one typical example is an edible natural material contained in palm oil, butter and nuts, and is also called octylic acid or caprylic acid. Specifically, the octanoic acid is a saturated normal-chain fatty acid that has carbon number 8, is slightly soluble in water, and is expressed by the molecular formula CH₃(CH₂)₆COOH with the molecular weight of 144.21, the melting point of 16.5 degrees cntigrade, the boiling point of 237 degrees centigrade. The octanoic acid is an edible natural material contained in palm oil and butter, and has been used in a drug applied to dyspeptics, for example.

Decanoic acid as another typical example is also called decylic acid or capric acid. Specifically, the decanoic acid is a saturated normal-chain fatty acid that has carbon number 10, is slightly soluble in water, and is expressed by the molecular formula CH₃(CH₂)₈COOH with the molecular weight of 172.27, the melting point of 31.4 degrees centigrade, the boiling point of 269 . degrees centigrade. The decanoic acid is a natural material contained in palm oil and hair fat.

The inventors conducted trials using tiger puffer to test whether the octanoic acid adversely affects growth of fish. Two trial groups were prepared; Le., one group fed the basal diet supplemented with 0.5 % of octanoic acid (5g of octanoic acid added for 1 kg of diet), and the other group fed the basal diet alone. Trial conditions were that 50 pieces of tiger puffer for each group were put in a 1-t water tank, and the puffs were fed twice per day, i.e., in the morning and evening. A bleeding trial period was 45 days and the water temperature was 25+-1 degrees centigrade during the trial period.

The average weight for each group was 34 g at the start of the trial period and 95 g at the end. Values of the body length, condition factor, weight gain and feed efficiency also showed similar differences between the start and the end of the trial period. Thus, adverse effects on the puffer growth were not found. Bleeding of those puffs was continued for succeeding seven months, and there were no died fish. As a result of analyzing the content of octanoic acid in the edible muscle of the puffs in an ordinary manner, no octanoic acid was detected. For human beings, it is reported that the absorption path of medium-chain fatty acids into the body is simpler than that of long-chain fatty acids, and the medium-chain fatty acids are quickly absorbed and promptly utilized as energy sources, resulting no appreciable accumulation in the body. Based on such properties, medium-chain fatty acids are used as energy sources for patients suffering digestive tract diseases. The above result suggests that medium-chain fatty acids can be utilized as energy sources and are hardly accumulated in the fish body as with human beings. If medium-chain fatty acids are accumulated in the fish body, the amount of the medium-chain fatty acids accumulated would be so small that no adverse effects act on persons who have eaten the fish.

The use of a natural physiologically active material effective on fish parasitic diseases, according to the present invention is not particularly limited in the form, can be employed as being added to a drug solution for bath treatment and fish food to give them an antiparasitic activity, an antibacterial activity and/or an antivirus activity. The composition and manufacture method of the fish food according to claim 5 of the present invention is not particularly limited, but may be optionally selected so long as the fish food contains fatty acids having carbon number 6 to 12.

The natural physiologically active material used according to the present invention is expected to be effective on fish parasitic diseases, when added to basal diet. The fish food and the fish food additive according to claim 5 of the present invention are not particularly limited in composition and manufacture method so long as the final product contains fatty acids having carbon number 6 to 12. When the fatty acids having carbon number 6 to 12 are added to fish food in the form of a solution, granules or tablets, the fish food preferably contains the fatty acids in amount of 0.125 - 2 weight % with respect to the total weight.

The fish food and the fish food additive according claim 5 of to the present invention give rise no adverse effects such as a reduction in feed intake, growth and so forth. The fatty acids having carbon number 6 to 12 are added to fish food in some stage of the food manufacturing process, which is appropriate from the technical point of view, so that the fatty acids having carbon number 6 to 12 are evenly distributed in the final product.

An immersing solution for use in the present invention is not particularly limited so long as the solution contains fatty acids having carbon number 6 to 12. When the immersing solution is prepared by dissolving the fatty acids having carbon number 6 to 12 in the form of a solution, granules or tablets, the solution concentration is preferably adjusted to fall in the range of 80 - 200 ppm.

### BRIEF DESCRIPTION OF THE DRAWINGS

Fig. 1 is a graph showing the efficacy of fatty acids on elimination of oncomiracidia and larvae of *Heterobothrium okamotoi*.
Fig. 2 is a graph showing the efficacy of fatty acids on elimination of theronts) of *Cryptocaryon irritans*.
Fig. 3 is a graph showing survival comparison results of infected fish in the *Heterobothrium okamotoi* trial on tiger puffer in the case of n = 6.
Fig. 4 is a graph showing survival comparison results of normal fish in the *Heterobothrium okamotoi* trial on tiger puffer in the case of n = 8.
Fig. 5 is a graph showing comparison results of the number of parasites attaching to the infected fish died during the period of the *Heterobothrium okamotoi* trial on tiger puffer.
Fig. 6 is a graph showing comparison results of the number of parasites attaching to the infected fish survived at the end of the period (after 73 days) of the *Heterobothrium okamotoi* trial on tiger puffer.
Fig. 7 is a graph showing comparison results of the number of parasites attaching to the normal fish survived after 30 days in the *Heterobothrium okamotoi* trial on tiger puffer (n = 4 for each group).
Fig. 8 is a graph showing comparison results of the number of parasites attaching to the normal fish died during the period of the *Heterobothrium okamotoi* trial on tiger puffer.
Fig. 9 is a graph showing comparison results of the number of parasites attaching to the normal fish in the octanoic-acid added group survived at the end of the period (after 73 days) of the *Heterobothrium okamotoi* trial on tiger puffer.
Fig. 10 is a graph showing comparison results of the number of parasites *Cryptocaryon irritans* after 3 days from the start of challenge in the *Cryptocaryon irritans* trial on red sea bream.
Fig. 11 is a graph showing comparison results of the number of parasites in the *Cryptocaryon irritans* trial on red sea bream.
Fig. 12 is a graph showing comparison results of the number of parasites *Heteraxine beterocerca* in the field trial on amberjack (n = 20 for each group). Fig. 13 is a graph showing comparison results of the number of parasites *Benedenia seriolae* in the field trial on amberjack (n = 20 for each group).
Fig. 14 is a graph showing comparison results of the number of eggs of blood flukes (Paradeontacylix) per gill in the field trial on amberjack (n = 20 for each group).
Fig. 15 is a graph showing comparison results of the number of parasites *Heterobothrium okamotoi* in the vitamin C enhancement trial on tiger puffer (n = 5).
Fig. 16 is a graph showing comparison results of the number of parasites *Cryptocaryon irritans* in the vitamin C enhancement trial on red sea bream (n = 5 for each group at each sampling).
Fig. 17 is a graph showing survival comparison results in the vitamin C enhancement trial on red sea bream.
Fig. 18 is a graph showing results of the number of parasites *Cryptocaryon* irritans in the efficacy comparison trials of octanoic acid and monoglyceride and triglyceride thereof on red sea bream (n = 5 for each group at each sampling).
Fig. 19 is a graph showing an antibacterial activity of fatty acids against *Vibrio* anguillarum.
Fig. 20 is a graph showing an antibacterial activity of fatty acids against *Edwardsiella* tarda.
Fig. 21 is a graph showing an antibacterial activity of fatty acids against Enterococcus seriolicida.
Fig. 22 is a graph showing fed diets and mortality changes in the bleeding trial on spotted plaice.
Fig. 23 is a graph showing mortality change in the Kuchijirosho challenge trial tiger puffer.

### BEST MODE FOR CARRYING OUT THE INVENTION

Details of the present invention will be described below in connection with Examples. It is to be understood that the Examples should not be construed in a way limiting the scope of the present invention.

### Example 1

### (Purpose)

Larvae of parasites *Heterobothrium okamotoi* were immersed in seawater in which octanoic acid was dissolved. As a result, it was observed that the larvae died due to the occurrence of structural damages (such as vacuolation). Octanoic acid is an edible natural material contained in palm oil, butter and nuts, and has been used in a drug applied to dyspeptics, for example.

In consideration of the above result, the following trials were conducted. In Trial 1, the efficacy of fatty acids, primarily medium-chain fatty acids, against parasites was investigated by *in vitro* assay. The parasites tested were *Heterobothrium okamotoi* that belongs to *Plathelminthes* and parasitizes to tiger puffer, and *Cryptocaryon irritans* that belongs to *Protozoa* and infects to many kinds of fish with remarkable damages. In Trial 2, the efficacy of oactanoic acid against the following parasites by *in vitro* assay; *Bivagina* tai that belongs to *Plathelminthes* and parasitizes to red sea bream, *Heteraxine heterocerca* parasitizing to young yellowtail, *Benedenia seriolae* causing a serious problem in young yellowtail and flounder, *Kudoa* that belongs to *Protozoa* and parasitizes in the muscle, heart, etc. with damages in value of edible fish, and *Caligus* that belongs to *Arthropoda* and gives rise to remarkable damages in salmons and many other marine fish. In Trials 3, 4 and 5, the efficacy of octanoic acid against parasites was investigated by adding octanoic acid to fish food to be fed orally. More specifically, in Trial 3, the efficacy against *Heterobothrium okamotoi* in tiger puffer was studied. In Trial 4, the efficacy against *Cryptocaryon irritans* in red sea bream was studied. In Trial 5, the efficacy against *Heteraxine heterocerca, Benedenia seriolae* and blood flukes (Paradeontacylix) in amberjack was studied. Further, in trials 6 and 7 it was investigated whether the efficacy is enhanced or not by adding vitamin C to the octanoic-acid added food in a greater amount than contained in ordinary fish food. More specifically, in Trial 6, the enhancement effect of vitamin C was studied on tiger puffer. In Trial 7, the enhancement effect of vitamin C was studied on red sea bream was studied. In Trial 8, it was investigated whether monoglyceride and triglyceride of octanoic acid act against *Cryptocaryon irritans* in red sea bream as effective as free octanoic acid was studied. Moreover, in Trials 9 and 10, it was investigated whether immersing fish in seawater added with octanoic acid is effective against parasites. More specifically, in Trial 9, the efficacy on fish was studied in
the case of immersing tiger puffer in seawater added with octanoic acid. In Trial 10, the efficacy in eliminating *Cryptocaryon irritans* was studied in the case of immersing tiger puffer infected with *Cryptocaryon irritans* in seawater added with octanoic acid.

### [Trial 1]

### (Method)

Fatty acids having carbon number C2, C4 and C6 - C10 were each dissolved in seawater at 1 mM concentration, and oncomiracidia of *Heterobothrium okamotoi* and larvae attaching to the gill were immersed in the seawater solutions. After 2 hours from the start of immersion, the efficacy of the fatty acids was investigated by counting the number of dead oncomiracidia and the number of larvae dropping down from the gill. More than 30 individuals of parasites were transferred to each group.

For *Cryptocaryon irritans*, fatty acids having carbon number C2, C4 and C6 - C10 were each dissolved in seawater at 0.5 mM concentration, and theronts of *Cryptocaryon irritans* were immersed in the seawater solutions. After 1 hour from the start of immersion, the efficacy of the fatty acids was investigated by counting the number of dead theronts. More than 200 individuals of parasites were transferred to each group.

For any of the groups, the trial was made under condition of 20 degrees centigrade.

### (Results and Discussion)

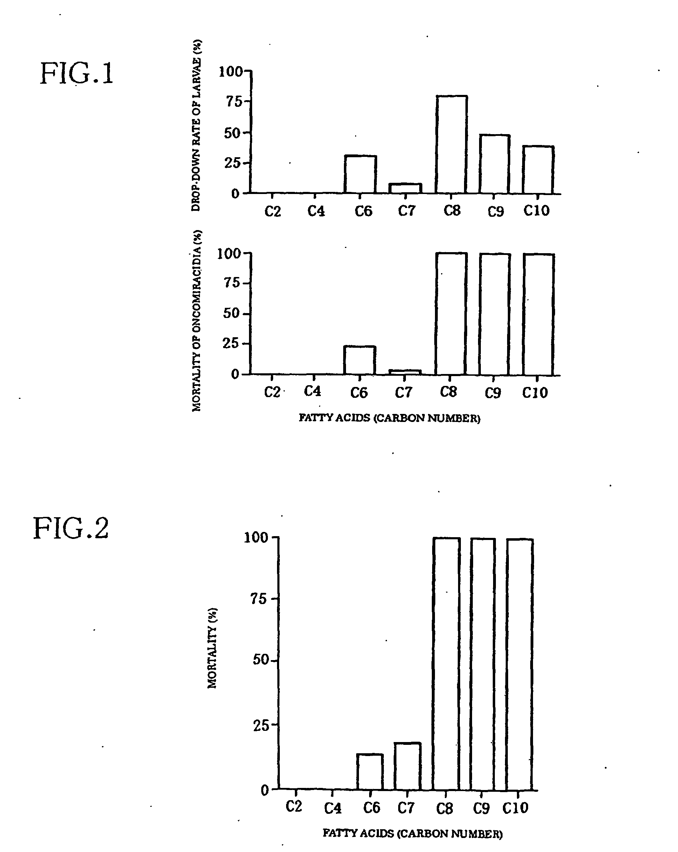

It was observed that larvae of *Heterobothrium okamotoi* were apparently atrophied and dropped down from the gill in the seawater solutions of C6 to C10. Of the seawater solutions of C6 to C10 showing the efficacy, the highest drop-down rate was obtained with C8 (see Fig. 1). Similarly, dead individuals of the oncomiracidia of *Heterobothrium okamotoi* were observed in the seawater solutions of C6 to C10. In particular, mortality of the oncomiracidia in the seawater solutions of C6 to C10 was 100 %. Among these solutions, the dead individual emerged earliest in C8 (Fig. 1).

For *Cryptocaryon irritans*, dead individual emerged in C6 to C10. As with the case of *Heterobothrium okamotoi*, C8 to C10 showed a high antiparasitic activity and provided mortality of 100 %. Among these solutions, the dead individual emerged earliest in C8 (Fig. 2).

From the above results, it was proved that medium-chain fatty acids were effective against parasites. Of the medium-fatty fatty acids, those having carbon number C8 to C10 were more effective, and that having carbon number C8 was most effective. The fatty acid having carbon number C12 was so low in solubility that the trial could not be made under the same condition. However, a similar activity was found even at a low concentration.

### [Trial 2]

### (Methods)

800 PPM of octanoic acid was added to seawater and dissolved therein maximally. In this seawater solution, *Bivagina* tai larvae sampled from the gill of red sea bream, *Heteraxine heterocerca* larvae sampled from the gill of young yellowtail, *Benedenia seriolae* adults sampled from the body surface of young yellowtail, *Kudoa shiomitsui* spores sampled from the heart of tiger puffer, and Pseudocaligus fugu adults sampled from the body surface of tiger puffer were immersed under condition of 20 degrees centigrade, followed by observation. As control, another group was managed using seawater alone. More than 30 individuals of parasites were transferred to each group.

### (Results and Discussion)

It was observed in the octanoic-acid added group that Biv*agina, Heteraxine* and *Benedenia* became contracted apparently. For any of the parasites, the contraction occurred in approximately 10 minutes. For *Kudoa*, a burst image of the spores was observed in approximately 1 hour. For (*Caligus*, no atrophy Pseudocaligus no contraction) was observed, but a reduction in swimming activity was found after approximately 1 hour. On the other hand, no changes of the parasites were found in control.

From the results of Trials 1 and 2, it was elucidated that octanoic acid has an effect in eliminating parasites quite different from the classification point of view, such as flatworms, arthropods and protozoa. It is therefore thought that octanoic acid will develop its efficacy against all kinds of fish parasites. Further, it is thought from the results of Trial 1 that the medium-chain fatty acids other than octanoic acid also similarly develop an effect in eliminating a variety of parasites.

### [Trial 3]

### (Methods)

Fish tested: Tiger puffer weighing approximately 133.6 g in average.
Trial groups: Two groups, i.e., an octanoic-acid added group in which fish were fed diets added with octanoic acid, and a control group in which fish were fed diets not added with octanoic acid. The feeding rate was 1 % of fish weight / day for each group.
Trial diets: The diets added with octanoic acid were prepared by spraying 2.5 g of octanoic acid to 1 kg of commercially available pellets for tiger puffer. The control group fish were fed the commercially available pellets for tiger puffer.
Challenge trial: Each 12 pieces of fish (normal fish) from among a shoal of fish, in which no diseases such as *Heterobothrium okamotoi* infection were found, were put in a 100-liter water tank, and were fed the respective trial diets. After 7 days, 6 pieces of fish (infected fish), which had been infected in advance with approximately 100 individuals of *Heterobothrium okamotoi*, were added to each trial group. Then, bleeding was continued for 73 days while feeding the trial diets. In this trial, the parasites *Heterobothrium okamotoi* in the infected fish were in the stage of adult. After 30 days from adding the infected fish, 4 pieces of normal fish were sampled from each group, and the number of parasites attaching to the gill was counted. During the trial period, the water temperature was in the range of 16.5 - 20.9 degrees centigrade and the water was exchanged at 20 cycles/day.

Evaluation was made by comparing mortality of the fish in respective groups during the trial period, and by counting the number of parasites attaching to the gill of the infected fish in each group for investigation of the remedy efficacy and the infection preventable efficacy.

### (Results and Discussion)

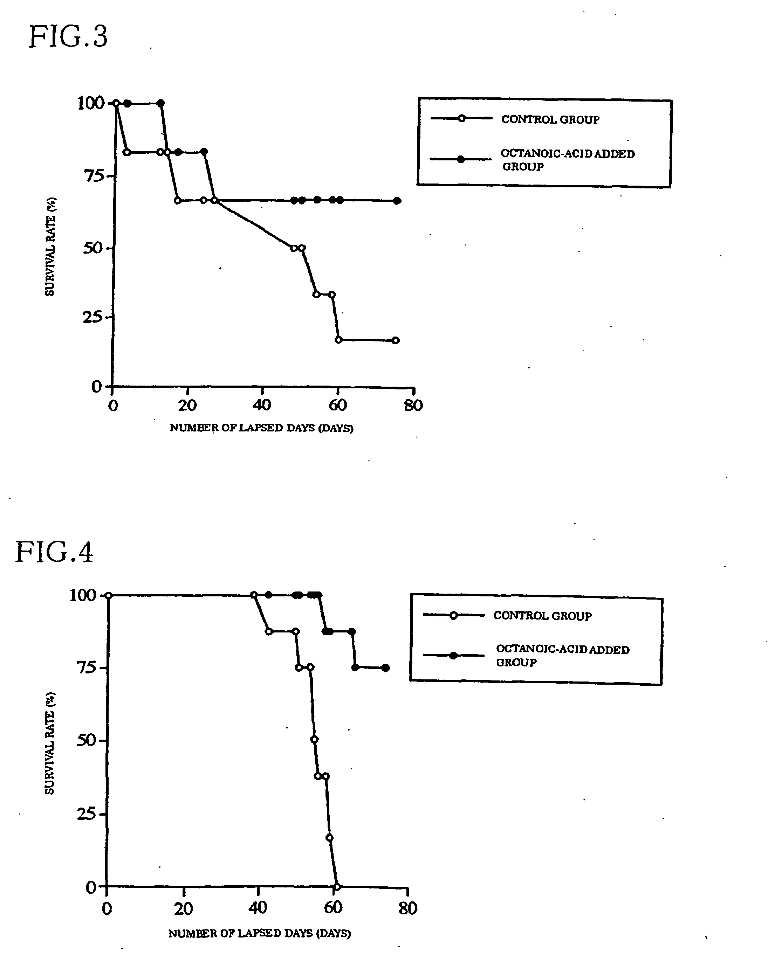

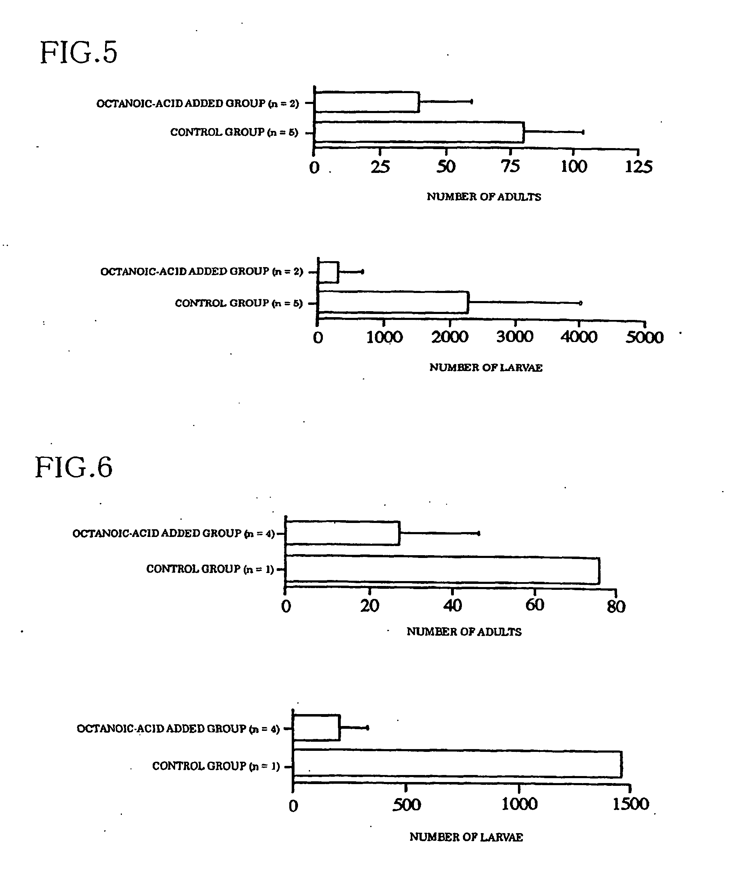

The survival rate of the infected fish at the end of the trial was 66.7 % in the octanoic-acid added group and 16.7 % in the control group (Fig. 3). The efficacy of octanoic acid was thus confirmed. The number of parasites attaching to the infected fish in the octanoic-acid added group, which were survived at the end of the trial, was apparently reduced in both adults and larvae as compared with the control group (Fig. 6). A similar tendency was also confirmed regarding the number of dead infected fish (Fig. 5). The approximately 100 individuals of adults attaching to each infected fish at the start of the trial were reduced to approximately 27 individuals at the end of the trial in each of the survived fish in the octanoic-acid added group. These results showed that octanoic acid is effective in eliminating adults of *Heterobothrium okamotoi* and hence effective in remedying the *Heterobothrium okamotoi* infection. Drugs such as hydrogen peroxide and formalin are not confirmed as to their efficacy in eliminating the adults, and cannot prevent propagation of the next generation. For this reason, when drugs such as hydrogen peroxide and formalin are used, it would be impossible to completely prevent the infection of *Heterobothrium okamotoi* and remedy the infected disease unless drug treatment has to be repeated frequently.

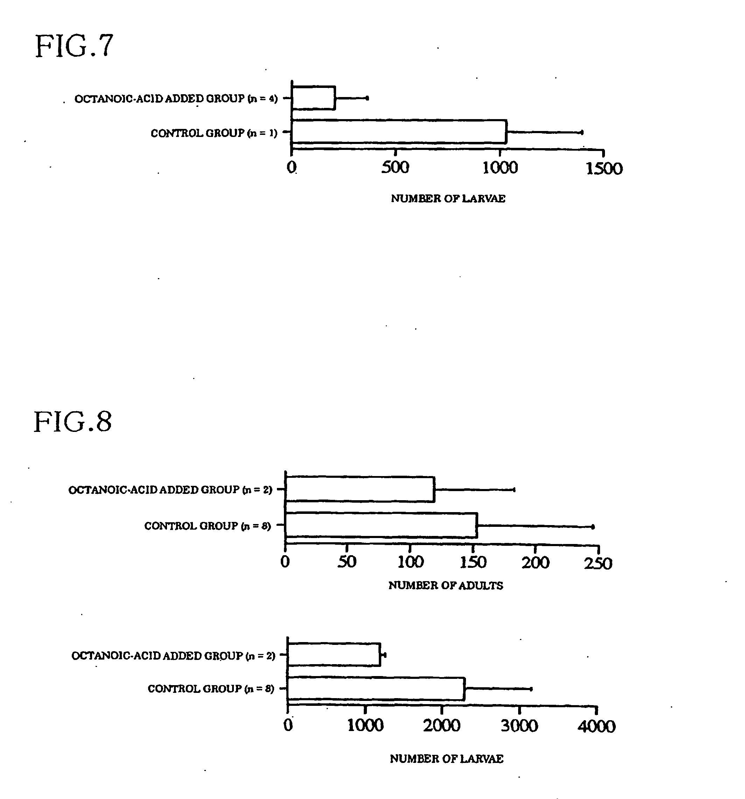

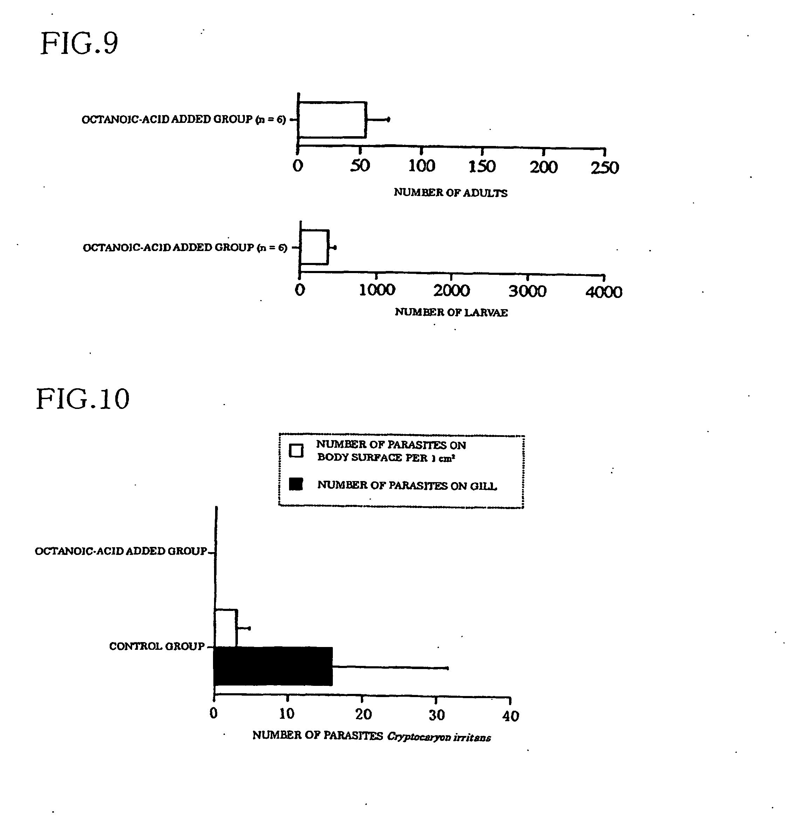

The survival rate of the normal fish was 75 % in the added group and 0 % in the control group (Fig. 4). The efficacy of octanoic acid was thus also confirmed as with the infected fish. The number of parasites attaching to the normal fish in the added group after 30 days was apparently reduced as compared with the control group, and therefore the infection preventable efficacy was confirmed (Fig. 7). The numbers of the parasites were also apparently reduced in both the survived fish after the end of the trial and the fish died during the trial period (Figs. 8 and 9). From the above results, the remedy efficacy and the infection preventable efficacy on *Heterobothrium okamotoi* infected fish were confirmed when octanoic acid was added to diets for oral administration to fish. It was further estimated that the similar efficacy on the fish parasitic diseases is also expectable by addition of other fatty acids having carbon number C6 to C12 than octanoic acid.

### [Trial 4]

### (Methods)

Fish tested: Red sea bream weighing approximately 9.8 g in average.
Trial groups: Two groups, i.e., an octanoic-acid added group in which fish were fed diets added with octanoic acid, and a control group in which fish were fed diets not added with octanoic acid. The feeding rate was 3 % of fish weight / day for each group.
Trial diets: The diets added with octanoic acid were prepared by spraying 5 g of octanoic acid to 1 kg of commercially available pellets for red sea bream. The control group fish were fed the commercially available pellets for red sea bream.
Challenge trial: Each 30 pieces of fish from among a shoal of fish, in which no diseases such as *Cryptocaryon irritans* infection were found, were put in a 100-liter water tank, and were fed the respective trial diets. After 5 days, approximately 2000 individuals of (oncomiracidia theronts) of *Cryptocaryon irritans* were put in each water tank to infect the fish while water flow was stopped for 1 hour. Then, bleeding was continued for 15 days. During the trial period, the water temperature was in the range of 22.5 - 23.2 degrees centigrade and the water was exchanged at 20 cycles/day.

Evaluation was made by sampling 5 pieces of fish from each group after 3 days from the start of the challenge, comparing the number of parasites attaching to the gill and the body surface of each infected fish, and comparing mortality of the fish during the trial period between the respective groups.

### (Results and Discussion)

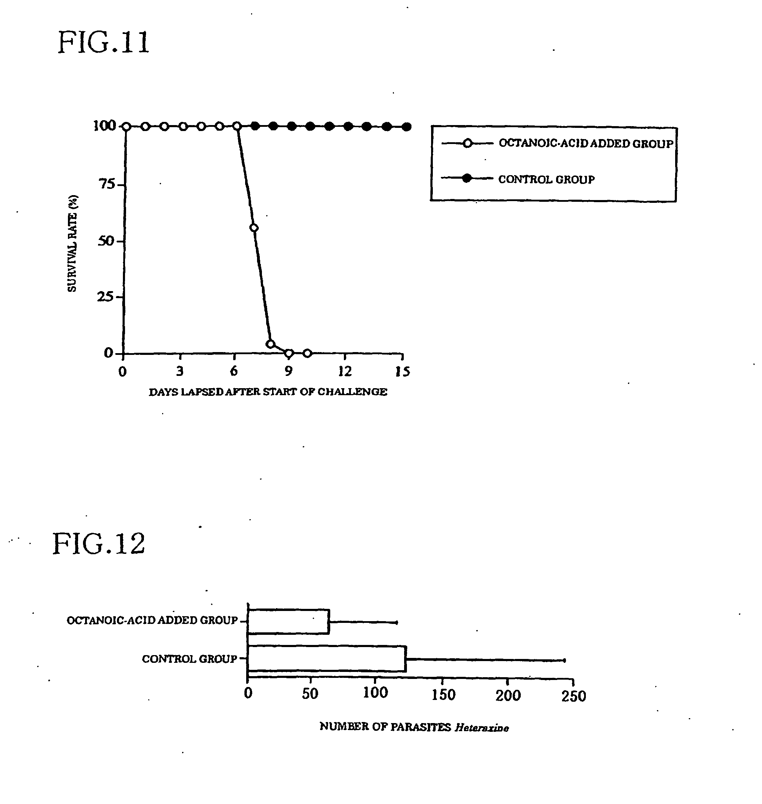

In the fish sampled from the octanoic-acid added group after 3 days from the challenge, no parasites were found, but in the control group, the parasites were found (Fig. 10). After that, in the control group, dying of the fish began at the 7-th day, and all the fish were dead at the 8-th day (Fig. 11). The number of the parasites attaching those dead fish in the control group was approximately 800 in the gill and approximately 20 per 1 cm2 of the body surface. Thus, the parasites apparently propagated. On the other hand, in the octanoic-acid added group, no fish died and no parasites were found even at the end of the trial. From the above results, it was evident that the infection and propagation of *Cryptocaryon irritans* can be prevented by adding octanoic acid to diets for oral administration to fish.

### [Trial 5]

### (Methods)

Fish tested: Amberjack weighing approximately 117 g in average.
Trial groups: Two groups, i.e., an octanoic-acid added group in which fish were fed diets added with octanoic acid, and a control group in which fish were fed diets not added with octanoic acid. The feeding rate was 3 % of fish weight / day for each group.
Trial diets: The diets added with octanoic acid were prepared by spraying 8 g of octanoic acid to 1 kg of commercially available pellets for amberjack. The control group fish were fed the commercially available pellets for amberjack.
Trial: The trial was conducted using off-shore net pens of 10 x 10 x 9 m placed in the field sea. Each 15000 pieces of fish for each group were treated with a parasiticide containing commercially available hydrogen peroxide as a main ingredient, and then put in each net pen. At this time, it was confirmed that *Heteraxine heterocerca* parasitizing to the gill and *Benedenia seriolae* parasitizing on the body surface were eliminated, by investigating 20 pieces of fish for each group. It was also simultaneously confirmed that blood flukes (Pradeontacylix) were present in the fish, by investigating the presence of eggs in the gill filaments. Then, bleeding was continued for 9 days while feeding the trial diets. The water temperature during the trial period was in the range of 18.5 - 20.0 degrees centigrade.

Evaluation was made by sampling 20 pieces of fish from each group after 9 days from the start of the bleeding, and counting and comparing the number of *Heterazme heterocerca* in the gill of each sampled fish, the number of *Benedenia seriolae* on the body surface, and the number of eggs of blood flukes (Paradeontacylix) in the gill filaments.

### (Results and Discussion)

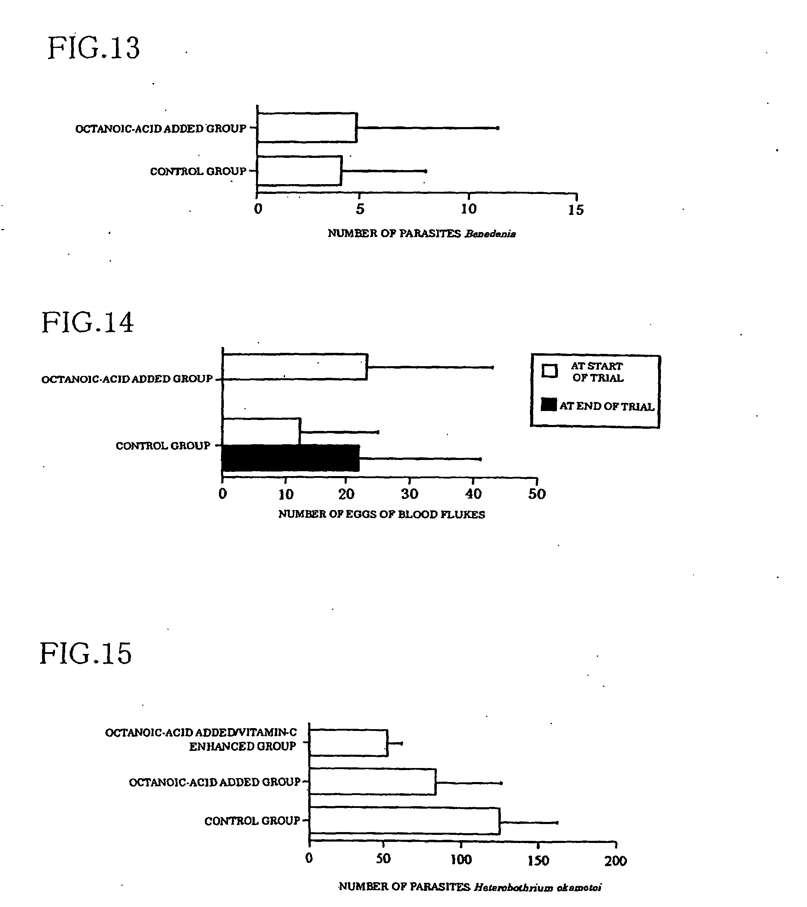

Immediately after the drug bath treatment, no *parasites Heteraxine heterocerca* and *Benedema seriolae* were found in both groups. The number of *Heteraxine heterocerca* in the sampled fish at the end of the bleeding was apparently fewer in the ocatnoic-acid added group than in the control group, and the efficacy of ocatnoic acid was confirmed (Fig. 12). In contrast, no appreciable difference in the number of *Benedenia seriolae* was found between both the groups (Fig. 13). The eggs of blood flukes (Paradeontacylix) were observed in both the groups at the start of the trial, and the presence of blood flukes (Paradeontacylix) in fish was confirmed. In the fish sampled at the end of the bleeding, the eggs of blood flukes (Paradeontacylix) were observed in the control group, but no eggs of blood flukes were observed in the octanoic-acid added group. It was concluded that octanoic acid is effective in eliminating the blood flukes (Fig. 14).

From the results of Trials 3, 4 and 5, it was confirmed that the remedy efficacy and the infection preventable efficacy on the parasitic infections were apparently developed by adding octanoic acid to diets for oral administration to fish. Since each efficacy was confirmed in several species of fish different in classifications, such as tiger puffer, red sea bream and amberjack, it is thought that octanoic acid will develop its efficacy on all species of fish. In human beings, medium-chain fatty acids are quickly absorbed after administration, and then utilized as energy sources. It is also known that the medium-chain fatty acids appear in blood in the course until being finally utilized as energy sources. In fish, it is similarly estimated that the medium-chain fatty acids are quickly absorbed after administration, and then appear in blood in the course until being finally utilized as energy sources. *Heterobothrium okamotoi, Cryptocaryon irritans* and *Heteraxine heterocerca*, against which the efficacy of octanoic acid was confirmed with in vivo trials, are reported to intake nutrients from host blood. Blood flukes (Paradeontacylix) parasitize in host blood vessels, and therefore are always in contact with host blood. On the other hand, *Benedenia seriolae*, against which the efficacy of octanoic acid was not confirmed, is reported to parasitize on the body surface and intake nutrients from mucus. Accordingly, it is estimated that the antiparasitic activity of the medium-chain fatty acids, confirmed with in vivo trials, is developed through the mechanism in which the applied fatty acids appear in blood and parasites intake the host blood containing the fatty acids or contact with the same.

### [Trial 6]

### (Methods)

Fish tested: Tiger puffer weighing approximately 50.0 g in average.
Trial groups: Three groups, i.e., an octanoic-acid added group in which fish were fed diets added with octanoic acid, an octanoic-acid added/vitamin-C enhanced group in which fish were fed diets added with both octanoic acid and vitamin C, and a control group in which fish were fed diets not added with octanoic acid. The feeding rate was 2 % of fish weight / day for each group.
Trial diets: The diets added with octanoic acid were prepared by spraying 5 g of octanoic acid to 1 kg of commercially available pellets for tiger puffer. The diets added with both octanoic acid and vitamin C were prepared by spraying 200 mg of vitamin C, dissolved in water, to 1 kg of the above diets added with octanoic acid. The control group fish were fed the commercially available pellets for tiger puffer.
Challenge trial: Each 5 pieces of fish from among a shoal of fish, in which no diseases such as parasitic infections were found, were put in a 100-liter water tank, and were fed the respective trial diets. After 7 days, approximately 750 individuals of oncomiracidia of *Heterobothrium okamotoi* were put into each water tank to infect the fish while water flow was stopped for 1 hour. The infection procedure was repeated every other day 5 times in total under the same condition. Then, bleeding was continued for 15 days after the first infection while feeding the trial diets. During the trial period, the water temperature was 25+- 1 degrees centigrade and the water was exchanged at 20 cycles/day.

Evaluation was made by sampling all the fish under trial in each group after 15 days from the first challenge, comparing the number of the parasites attaching to the gill of each fish, and comparing mortality of the fish during the trial period between the respective groups.

### (Results and Discussion)

The number of the parasites was apparently fewer in the octanoic-acid added group than in the control group, and the efficacy of addition of octanoic acid was reproduced (Fig. 15). The number of the parasites in the octanoic-acid added/vitamin-C enhanced group was even fewer than in the octanoic-acid added group for which the efficacy of octanoic acid was confirmed. From the above results, it was proved that the efficacy of octanoic acid against the parasites *Heterobothrium okamotoi* was further increased by adding both octanoic acid and vitamin C to the diets in a combined manner, the vitamin C being in a greater amount than contained in ordinary fish food.

### [Trial 7]

### (Methods)

Fish tested: Red sea bream weighing approximately 15.8 g in average.
Trial groups: Three groups, i.e., an octanoic-acid added group in which fish were fed diets added with octanoic acid, an octanoic-acid added/vitamin-C enhanced group in which fish were fed diets added with both octanoic acid and vitamin C, and a control group in which fish were fed diets not added with octanoic acid. The feeding rate was 3 % of fish weight / day for each group.
Trial diets: The diets added with octanoic acid were prepared by spraying 2.5 g of octanoic acid to 1 kg of commercially available pellets for red sea bream. The diets added with both octanoic acid and vitamin C were prepared by spraying 200 mg of vitamin C, dissolved in water, to 1 kg of the above diets added with octanoic acid. The control group fish were fed the commercially available pellets for red sea bream.
Challenge trial: Each 30 pieces of fish from among a shoal of fish, in which no diseases such as *Cryptocaryon irritans* infection were found, were put in a 100-liter water tank, and were fed the respective trial diets. After 5 days, approximately 2000 individuals of theronts of *Cryptocaryon irritans* were put into each water tank to infect the fish while water flow was stopped for 1 hour. Then, bleeding was continued for 60 days. During the trial period, the water temperature was 24 +- 1 degrees centigrade and the water was exchanged at 20 cycles/day.

Evaluation was made by sampling 5 pieces of fish from each group after 18 and 31 days from the start of the challenge, comparing the number of the parasites attaching to the gill of each fish, and comparing mortality of the fish during the trial period between the respective groups.

### (Results and Discussion)

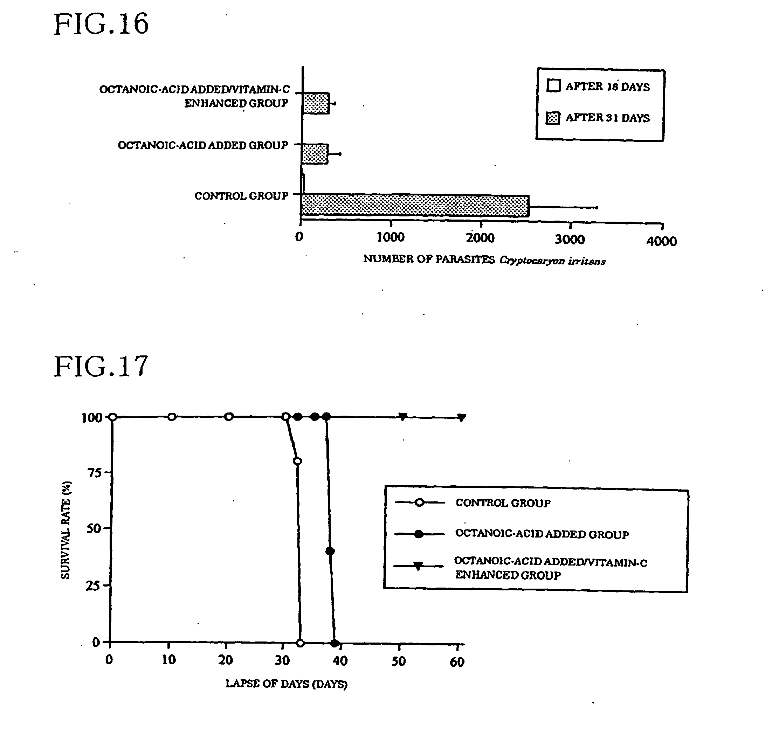

In the fish sampled after 18 and 31 days from the start of the challenge, the number of the parasites was apparently fewer in the octanoic-acid added group than in the control group, and the efficacy of addition of octanoic acid was reproduced (Fig. 16). The fish in the octanoic-acid added group were all dead as with the fish in the control group, but the time at which the fish began dying was later in the octanoic-acid added group than in the control group. The efficacy of addition of octanoic acid was thus confirmed (Fig. 17). The numbers of the parasites after 18 and 31 days were comparable between the octanoic-acid added/vitamin-C enhanced group and the octanoic-acid added group. In the octanoic-acid added/vitamin-C enhanced group, however, no fish died until the end of the trial, and no parasites were found in the gill and on the body surface of the survived fish. Further, no cysts of the parasites were found in the water tank. It was therefore thought that the parasites were remedied in the fish of the octanoic-acid added/vitamin-C enhanced group.

From the above results, it was proved that the efficacy of octanoic acid against the parasites *Cryptocaryon irritans* was further increased by adding both octanoic acid and vitamin C to the diets in a combined manner, the vitamin C being in a greater amount than contained in ordinary fish food. In fish, studies have been made on the immunological enhancement of vitamin C and the efficacy of vitamin C is confirmed. Stated otherwise, the antiparasitic activity of octanoic acid was enhanced by the combined use of octanoic acid and the material showing the immunological enhancement. It is also estimated that such a combined effect is similarly developed against bacteria and viruses described later.

### [Trial 8]

### (Methods)

Fish tested: Red sea bream weighing approximately 15.8 g in average.
Trial groups: Four groups, i.e., an octanoic-acid added group in which fish were fed diets added with octanoic acid, a monoglyceride added group in which fish were fed diets added with monoglyceride of octanoic acid, a triglyceride added group in which fish were fed diets added with triglyceride of octanoic acid, and a control group in which fish were fed diets added with neither octanoic acid nor glycerides. The feeding rate was 3 % of fish weight / day for each group.
Trial diets: The diets added with octanoic acid were prepared by spraying 2.5 g (17.5 mM with respect to the diet weight) of octanoic acid to 1 kg of commercially available pellets for red sea bream. Monoglyceride and triglyceride of octanoic acid were added to the diets at the amount equal to 17.5 mM residual radicals of octanoic acid. Specifically, the diets added with monoglyceride and triglyceride were prepared respectively by spraying 3.8 g of monoglyceride and 2.7 g of triglyceride to 1 kg of the commercially available pellets for red sea bream. The control group fish were fed the commercially available pellets for red sea bream.
Challenge trial: Each 10 pieces of fish from among a shoal of fish, in which no diseases such as *Cryptocaryon irritans* infection were found, were put in a 100-liter water tank, and were fed the respective trial diets. After 5 days, approximately 2000 individuals of theronts of *Cryptocaryon irritans* were put into each water tank to infect the fish while water flow was stopped for 1 hour. Then, bleeding was continued for 31 days while feeding the respective trial diets. During the trial period, the water temperature was 24 +- 1 degrees centigrade and the water was exchanged at 20 cycles/day.

Evaluation was made by sampling 5 pieces of fish from each group after 18 and 31 days from the start of the challenge, and comparing the number of the parasites attaching to the gill of each fish.

### (Results and Discussion)

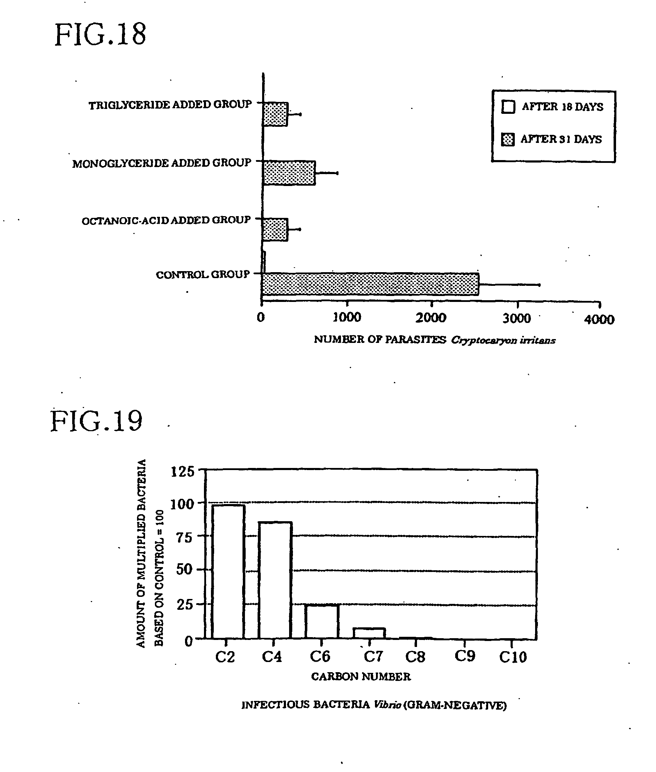

In the fish sampled after 18 and 31 days from the start of the challenge, the number of the parasites was apparently fewer in the octanoic-acid added group than in the control group, and the efficacy of addition of octanoic acid was reproduced (Fig. 18). The number of the parasites in each of the monoglyceride and triglyceride added groups was also apparently fewer than in the control group, and the efficacy of addition of monoglyceride and triglyceride was confirmed.

The inventors confirmed by *in vitro* assay that triglyceride of octanoic-acid showed no antibacterial activity against *Cryptocaryon irritans*, but monoglyceride of octanoic-acid showed an antibacterial activity though at a lower level than octanoic acid. In human beings, triglycerides of medium-chain fatty acids become free fatty acids in the intestinal tract under the action of lipase, and are carried to the liver via the portal vein. During such a transfer, the free fatty acids appear in blood. In fish, it is thought that triglycerides of medium-chain fatty acids become free fatty acids in the body fish and develop an antibacterial activity through the similar digestion process.

From the above results and phenomena, it was proved that monoglycerides and triglycerides of medium-chain fatty acids develop the efficacy against the parasites, along with bacteria and viruses described later, through metabolism in the fish body.

### [Trial 9]

### (Methods)

Each group, i.e., 130 g of tiger puffer, was immersed in 2-liter seawater containing octanoic acid at 100ppm,200ppm and 400ppm, followed by observation.

### (Results and Discussion)

In the 100 ppm group, after 60 minutes, a swimming activity of the fish was slightly lowered, but no abnormality was found in the fish. When returned to seawater again, the fish showed no abnormality. In the 200 ppm group, a swimming activity was apparently lowered after 30 minutes, and the fish began to lie down at 60 minutes. When returned to seawater again, the fish restored to a normal condition after approximately 30 minutes. In the 400 ppm group, the fish began to lie down at 3 minutes and became almost dying at 9 minutes. When returned to seawater again in the almost dying condition, the fish restored to a normal condition after approximately 60 minutes. The fish returned to seawater were observed for 7 days, and no abnormality was found. From the above results, it is thought that immersion treatment is practicable by ascertaining proper concentrations depending on the fish species.

### [Trial 10]

### (Methods)

300 g of tiger puffer infected with *Cryptocaryon irritans* was immersed in seawater containing 200 ppm of octanoic acid for 2 minutes, and then returned to seawater again. After 3 hours from return to seawater, the parasites *Cryptocaryon irritans* and their situations and numbers were observed.

### (Results and Discussion)

The number of the parasites attaching to the body surface was 261 / 2 cm². Of the individuals, the number of those that showed movement and seemed to be apparently still alive was 9. The number of the parasites attaching to the gill was 356 per branchial arch. Of the individuals, the number of those that showed movement and seemed to be apparently still alive was 193. The number of the parasites in tiger puffer investigated before the treatment was 193 / 2 cm² of body surface and 624 per branchial arch. Any of these parasites showed movement. From the above results, it is thought that immersing fish in seawater containing octanoic acid is also effective in killing the parasites *Cryptocaryon irritans* attaching to the fish.

### Example 2 (Reference Example)

### (Purpose)

The antibacterial activity of medium-chain fatty acids is known, but no reports regarding fish disease bacteria have been publicized. In Trial 11, the antibacterial activity of medium-chain fatty acids, primarily decanoic acid, against fish disease bacteria was investigated by *in vitro* assay. In Trial 12, the remedy efficacy and the infection preventable efficacy against the bacterial diseases were investigated by adding decanoic acid to diets for spotted plaice to be fed orally.

### [Trial 11]

### (Methods)

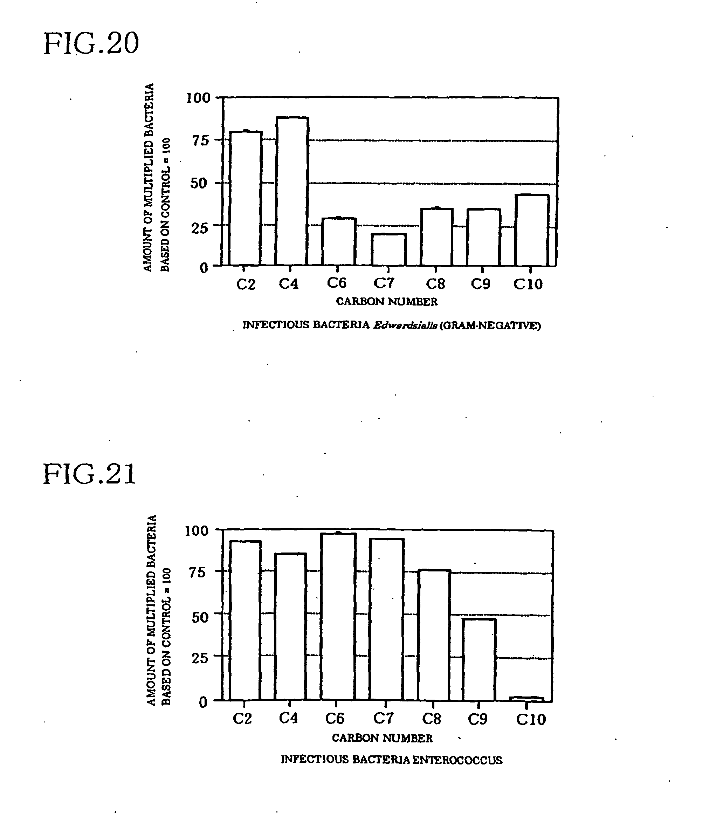

The turbidimetric analysis was employed. Fatty acids of C2, C4, C6-C10 were each dissolved in 2 ml of BHI liquid culture medium at 500 ppm concentration. A number 1 x 10⁵ of typical bacteria (*Vibrio anguillarum, Edwardsiella tarda* and *Enterococcus seriolicida*) causing the fish bacterial diseases were inoculated in each adjusted culture medium, followed by cultivation for 13 hours at 23 degrees centigrade. After the cultivation, turbidity of the culture medium was measured at 600 nm to determine the amount of multiplied bacteria. The trial was conducted two times. Results are shown in Fig. 19 (*Vibrio anguillarum*), Fig. 20 (*Edwardsiella tarda*), and Fig. 21 (*Enterococcus seriolicida*).

### (Results and Discussion)

Fatty acids of C6 to C10 apparently showed an antibacterial activity against *Vibrio anguillarum* and *Edwardsiella tarda*. Fatty acids of C9 and C10 showed an antibacterial activity against *Enterococcus seriolicida in* addition to *Vibrio anguillarum* and *Edwardsiella tarda*.

From the above results, it was proved that the fatty acids of C6 to C10 had an antibacterial activity against the fish bacteria. Of them, the fatty acids of C9 and C10 are expected to show an antibacterial activity against many fish bacteria. The fatty acid of C12 was so low in solubility that the trial could not be made under the same condition. However, a similar activity was found even at a low concentration.

### [Trial 12]

### (Methods)

Spotted plaice weighing approximately 60 g in average was employed in this trial. Each 270 pieces of spotted plaice were put in off-shore net pens of 4 x 4 x 2.5m, and bled for 100 days. The trial was conducted in two manners for the occurred bacterial disease; one in which exytetracycline (OTC), an antibiotic substance, was mixed in diets to be applied for 6 days at a rate of 0.5 g of 10 % OTC / kg of fish weight, and the other in which diets containing 0.5 % of decanoic acid were continuously fed. The number of dead fish and the fish situations were observed. The fish were fed twice per day, i.e., in the morning and evening. The water temperature during the trial period was in the range of 14 - 17 degrees centigrade.

### (Results and Discussion)

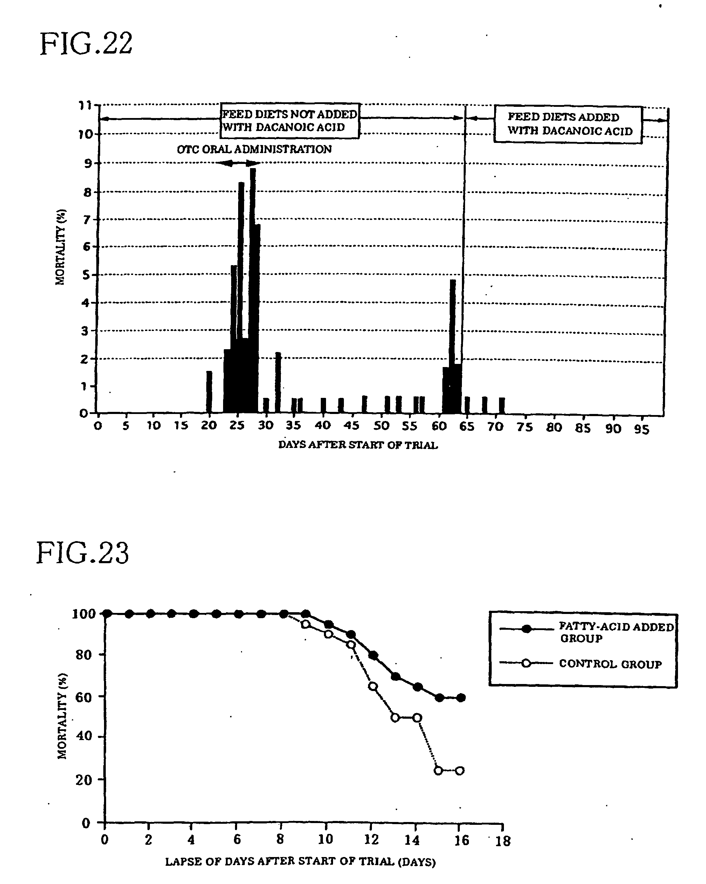

At the 20-th day from the start of the bleeding, *Vibrio* sp. infection and gliding bacterium infection occurred, and dead fish emerged. OTC was applied for 6 days from the 22-th to 27-th day from the start of the bleeding. After that, the number of dead fish was reduced, but complete remedy of the *Vibrio* sp. infection was not confirmed and dying of the fish still continued (Fig.22). From the 61-th day after the start of the trial, the number of dead fish due to the *Vibrio* sp. infection and the gliding bacterium infection increased again. The decanoic-acid added diets were fed from the 64-th day after the start of the trial. The number of dead fish was apparently reduced thereafter, and no dead fish were found after the 72-th day from the start of the trial. Further, fish suffering abrasion and bleeding due to the *Vibrio* sp. infection and the gliding bacterium infection were also not found. This satisfactory condition continued until the end of the bleeding.

From the above results, the remedy efficacy and the infection preventable efficacy on the fish bacterial diseases were confirmed by adding decanoic acid to diets for oral administration to fish. It was further estimated that the similar efficacy on the fish bacterial diseases is also expectable by addition of other fatty acids of C6 to C12 than decanoic acid. The above result also supports the efficacy of medium-chain fatty acids against parasites at in vivo level. Thus, it is thought that the applied medium-chain fatty acids appear in blood, and fish disease bacteria are affected upon contacting the blood. Considering the above mechanism, the efficacy with application at in vivo level is expectable in all species of fish on the bacterial diseases for which the efficacy was confirmed by *in vitro* assay.

### Example 3 (Reference Example)

### (Purpose)

The antivirus activity of medium-chain fatty acids is known, but no reports regarding oral administration for fish disease viruses have been publicized. In this Example, the inventors studied Kuchijirosho (snout ulcer disease) in tiger puffer which has brought about the biggest damages among the virus diseases. Since first reported in 1981, the occurrence of Kuchijirosho has been reported every year, and serious damages have been encountered. However, effective prevention and treatment methods have not yet been found. In view of the above situations, the inventors studied whether the efficacy against the occurrence of Kuchijirosho is developed by adding fatty acids of C6 to C12 to diets for tiger puffer.

### [Trial 13]

### (Methods)

Fish tested: Tiger puffer weighing approximately 27.7 g in average.
Trial groups: Two groups, i.e., a fatty-acid added group in which fish were fed diets added with fatty acids, and a control group in which fish were fed diets not added with fatty acids. The feeding rate was 2 % of fish weight / day for each group.
Trial diets: The diets added with fatty acids were prepared by spraying 10 g of fatty acids, consisting of C6, C8, C10 and C12 in equal amounts, to each 1 kg of commercially available pellets for tiger puffer. The control group fish were fed the commercially available pellets for tiger puffer.
Challenge trial: Each 20 pieces of fish from among a shoal of fish, in which no diseases such as Kuchijirosho infection were found, were put in a 100-liter water tank, and were fed the respective trial diets. After 14 days, a virus solution, prepared by diluting an undiluted solution at a rate of 10-8, was inoculated to each of all the fish. The virus solution was inoculated to the muscle in amount of 50 (m per fish. Then, bleeding was continued for 16 days from the start of the challenge. Mortality of the fish in the respective groups was compared. The water temperature during the trial period was 25 +1 degrees centigrade and the water was exchanged at 20 cycles/day.

### (Results and Discussion)

As shown in Fig. 19, the survival rate of the infected fish at the end of the trial was 60 % in the fatty-acid added group and 25 % in the control group (Fig.23). It was thus confirmed that the occurrence of Kuchijirosho can be prevented by oral administration of fatty acids to fish.

From the above results, it was proved that the remedy efficacy and the infection preventable efficacy on the fish virus diseases are expected by adding fatty acids of C6 to C12 to diets for fish. The above result also supports the efficacy of medium-chain fatty acids against parasites and bacteria at in vivo level. Thus, it is thought that the applied medium-chain fatty acids appear in blood, and fish disease viruses are affected upon contacting the blood. Considering the above mechanism, the efficacy with application at in vivo level is expectable in all species of fish on the viruses diseases for which the efficacy was confirmed by *in vitro* assay.

### Industrial Applicability

The present invention can provide the use of a natural physiologically active material effective on fish parasitic diseases.

Merely by, for example, employing the natural physiologically active material as a component of basal diets for fish or dissolving the material in a bath treatment solution to develop an antiparasitic, it becomes possible to bleed fish in aquaculture while avoiding the fish from dying with the fish parasitic diseases.

## Claims

1. Use of a physiologically active material containing at least one of fatty acids having carbon number 6 to 12 for the manufacture of a formulation for treating fish with parasitic diseases or protecting fish from parasitic diseases.

2. The use according to claim 1, wherein the at least one of fatty acids having carbon number 6 to 12 is selected from free fatty acids having carbon number 6 to 12, and salts, glycerides, esters and amides having residual radicals of the fatty acids having carbon number 6 to 12 in molecules thereof.

3. The use according to anyone of claims 1 or 2, wherein the at least one of fatty acids having carbon number 6 to 12 is selected from capric acid, caprylic acid and pelargonic acid.

4. The use according to anyone of claims 1 to 3, wherein the physiologically active material further contains a material having a vitamin C activity.

5. The use according to anyone of claims 1 to 4, wherein the formulation is a fish food or a fish food additive.

## Patentansprüche

1. Verwendung eines physiologisch wirksamen Materials, das wenigstens eine Fettsäure mit einer Kohlenstoffanzahl von 6 bis 12 enthält, für die Herstellung einer Formulierung für die Behandlung von Fisch mit parasitären Erkrankungen oder zum Schutz von Fisch vor parasitären Erkrankungen.

2. Verwendung nach Anspruch 1, wobei wenigstens eine der Fettsäuren mit einer Kohlenstoffanzahl von 6 bis 12 ausgewählt wird aus freien Fettsäuren mit einer Kohlenstoffanzahl von 6 bis 12 und Salzen, Glyzeriden, Estern und Amiden mit Radikalresten der Fettsäuren mit einer Kohlenstoffanzahl von 6 bis 12 in ihren Molekülen.

3. Verwendung nach einem der Ansprüche 1 oder 2, wobei die wenigstens eine der Fettsäuren mit einer Kohlenstoffanzahl von 6 bis 12 aus Caprinsäure, Caprylsäure und Pelargonsäure ausgewählt wird.

4. Verwendung nach einem der Ansprüche 1 bis 3, wobei das physiologisch wirksame Material weiterhin ein Material mit Vitamin-C-Aktivität enthält.

5. Verwendung nach einem der Ansprüche 1 bis 4, wobei die Formulierung ein Fischfutter oder ein Fischfutterzusatz ist.

## Revendications

1. Utilisation d'une matière physiologiquement active, contenant au moins l'un des acides gras ayant 6 à 12 atomes de carbone, pour la production d'un composé pour traiter le poisson présentant des maladies parasitaires ou protéger le poisson de maladies parasitaires.

2. Utilisation selon la revendication 1, dans laquelle l'au moins un acide gras ayant 6 à 12 atomes de carbone est choisi parmi les acides gras libres ayant 6 à 12 atomes de carbone et les sels, les glycérides les esters et les amides ayant des radicaux résiduels des acides gras ayant 6 à 12 atomes de carbone dans leurs molécules.

3. Utilisation selon l'une quelconque des revendications 1 et 2, dans laquelle l'au moins un acide gras ayant 6 à 12 atomes de carbone est choisi parmi l'acide caprique, l'acide caprylique et l'acide pélargonique.

4. Utilisation selon l'une quelconque des revendications 1 à 3, dans lequel la matière physiologiquement active contient en outre un matériau ayant une activité de vitamine C.

5. Utilisation selon l'une quelconque des revendications 1 à 47, dans lequel le composé est une nourriture pour poissons ou un aditif pour nourriture pour poisson.
